# EUROPÄISCHE PATENTSCHRIFT

(11) **EP 0 503 246 B1**
(45) Veröffentlichungstag und Bekanntmachung des Hinweises auf die Patenterteilung: **11.10.1995**
(21) Anmeldenummer: 92101054.2
(22) Anmeldetag: 23.01.1992
(51) Int. Cl.: C07C 209/48, C07C 209/52, C07C 255/66

(54) **Verfahren zur Herstellung von 3-Aminomethyl-3,5,5-trimethylcyclohexylamin**
Process for the preparation of 3-aminomethyl-3,5,5-trimethylcyclohexylamine
Procédé pour la préparation de la 3-aminométhyl-3,5,5-triméthylcyclohexylamine

(30) Priorität: 05.03.1991 DE 4106882
(43) Veröffentlichungstag der Anmeldung: 16.09.1992
(73) Patentinhaber: Degussa Aktiengesellschaft, 60311 Frankfurt (DE)
(72) Erfinder: Huthmacher, Klaus, Dr., W-6460 Gelnhausen (DE); Schmitt, Hermann, W-6451 Rodenbach (DE)

(56) Entgegenhaltungen:
- EP-A- 0 042 119
- EP-A- 0 394 968
- DE-A- 3 011 656

## Beschreibung

Die Erfindung richtet sich auf ein Verfahren zur Herstellung von 3-Aminomethyl-3,5,5-trimethylcyclohexylamin durch reduktive Aminierung von 1,3,3-Trimethyl-5-oxo-cyclohexan-carbonitril mit Ammoniak und Wasserstoff unter Verwendung eines Nickel- oder Kobalt-Raney-Katalysators.

3-Aminomethyl-3,5,5-trimethylcyclohexylamin, nachfolgend als Isophorondiamin bezeichnet, wird als Ausgangsprodukt zur Herstellung von Isophorondiisocyanat, als Aminkomponente für Polyamide und als Härter für Epoxidharze verwendet.

Bisher wird Isophorondiamin (IPDA) durch reduktive Aminierung von 1,3,3-Trimethyl-5-oxo-cyclohexancarbonitril, nachfolgend als Isophoronnitril (IPN) bezeichnet, in Gegenwart von Ammoniak und üblichen Hydrierkatalysatoren gewonnen. Das als Ausgangsverbindungen dienende Isophoronnitril ist durch Anlagerung von Cyanwasserstoff an Isophoron erhältlich - vgl. DE-Patentanmeldung P 39 42 371.9.

Nach dem Verfahren der DE-PS 12 29 078 werden Ammoniak und IPN im Molverhältnis von 10 bis 30 zu 1 eingesetzt, um IPDA zu gewinnen. Außer dem gewünschten IPDA entstehen aber in größerer Menge Nebenprodukte, wie insbesondere 3-Aminomethyl-3,5,5-trimethylcyclohexanol (=Isophoronaminoalkohol (IPAA)), 1,3,3-Trimethyl-6-aza-bicyclo-[3,2,1]-octan und Dihydroisophorylamin. Beispielhaft wird eine Ausbeute von bis zu 81,4 % IPDA angegeben, jedoch fehlen Reinheitsangaben. Die genannte Ausbeute erwies sich, wie von verschiedenen Seiten festgestellt wurde, als nicht reproduzierbar.

Im Bestreben, eine hohe Ausbeute an IPDA zu erhalten und den Zwangsanfall an IPAA zu minimieren, wurde gemäß DE-OS 30 11 656 das aus der DE-PS 12 29 078 bekannte Verfahren dahingehend abgeändert, daß in einer ersten Stufe IPN katalysatorfrei mit überschüssigem Ammoniak in 1,3,3-Trimethyl-5-iminocyclohexan-carbonitril überführt und dieses in einer zweiten Stufe zu IPDA hydriert wurde. In der 2. Stufe mußte ein erheblicher Überschuß an Ammoniak eingesetzt werden. Diese Arbeitsweise macht eine aufwendige Druckdestillation zwecks Rückgewinnung und Rezyklierung des Ammoniaks erforderlich. Beispielsgemäß wurde im Verfahren der DE-OS 30 11 656 trotz eines Mengenverhältnisses von etwa 5 kg Ammoniak pro 1 kg IPN nur eine Reaktionsausbeute von 83,7 % erzielt; über die Ausbeute an isoliertem IPDA und dessen Reinheit wurden keine Angaben gemacht.

Daß ein weiterer Bedarf bestand, die Verfahren der vorgenannten Dokumente zu verbessern, folgt aus der DE-OS 30 21 955. Gemäß Vergleichsbeispiel 1 der DE-OS 30 21 955 gelangt man trotz eines IPN/NH₃-Volumenverhältnisses Von 1 zu 10 im Verfahren analog DE-PS 12 29 078 nur zu einer IPDA-Ausbeute von 48 % - hierbei wurde ein nicht spezifizierter "handelsüblicher Katalysator" verwendet. Gemäß den Vergleichsbeispielen 2 und 3 der DE-OS 30 21 955, durchgeführt analog DE-OS 30 11 646, konnte eine Reaktionsausbeute von ca. 70 % bzw. 90 % ereicht werden; hierzu waren aber eine lange Reaktionszeit für die erste Stufe und ein IPN/NH₃-Volumenverhältnis von 1 zu 10 in der zweiten Stufe erforderlich. Zu dem Nachteil des hohen Ammoniaküberschusses kommt also noch eine wirtschaftlich bedeutsame Minderung der Raum-Zeit-Ausbeute.

Die lange Reaktionszeit für die erste Stufe - Iminbildung - im Verfahren der DE-OS 30 11 656 konnte gemäß DE-OS 30 21 955 dadurch reduziert werden, daß ein Iminbildungskatalysator eingesetzt wurde. Hierdurch wurde aber die Reaktionsführung aufwendiger und zudem konnte auf ein sehr hohes Ammoniak/IPN-Volumenverhältnis nicht verzichtet werden.

In den Verfahren der DE-OS 30 11 656 und 30 21 955 werden trägerfreie oder trägergebundene Co-, Ni-, Fe- und Edelmetallkatalysatoren, unter anderem auch Raney-Nickel und Raney-Kobalt, als geeignet bezeichnet. Auf die mögliche Mitverwendung katalytischer Mengen an Säuren und Ammoniumsalzen wird in der DE-OS 30 11 656 hingewiesen, ohne aber Einzelheiten über die Art der Verwendung zu offenbaren.

Das Verfahren der JP-A 62-123154 richtet sich auf die reduktive Aminierung von IPN zur Herstellung von IPDA, wobei versucht wurde, den erforderlichen Ammoniaküberschuß zu vermindern und auf die Vorreduktion des trägergebundenen Katalysators zu verzichten. Bei Verwendung der 1- bis 20-fachen, vorzugsweise 5- bis 10-fachen Molmenge Ammoniak, bezogen auf IPN, sowie Raney-Kobalt als Katalysator, einem Druck von 50 bis 150 bar und einer Temperatur von 50 bis 150 °C soll es nach dem genannten Verfahren möglich sein, IPDA in hoher Ausbeute zu gewinnen - in den Beispielen werden Ausbeuten zwischen 83 und 89 % im Reaktionsgemisch angegeben.

Die Anmelderin stellte bei der Nacharbeitung des Verfahrens der JP-A 62-123154 fest, daß trotz Erhöhung des Ammoniaküberschusses über den in dem japanischen Dokument angegebenen Wert fast keine Wasserstoffaufnahme stattfand, Raney-Kobalt allein also praktisch unwirksam ist.

Die EP-A 0 394 968 lehrt ebenfalls ein Verfahren zu reduktiven Aminierung von Carbonylverbindungen, darunter Isophoronnitril, woraus Isophorondiamin gebildet wird. Das Verfahren umfaßt die Verwendung eines Aminierungspromotors, vorzugsweise einer Hydoxylverbindung, Wasserentfernung, einen geringen H₂-Partialdruck bei der Iminbildung. Als Hydrierkatalysator dienen Raney-Katalysatoren, welche auch aktivitäts-/selektivitätssteigernde Dotierungselemente oder deren Verbindungen enthalten können; bevorzugt wird Cr- oder Mn-dotiertes Raney-Kobalt.

Aufgabe der vorliegenden Erfindung ist, das Verfahren zur Herstellung von Isophorondiamin aus Isophoronnitril dahingehend zu verbessern, daß auch mit den leicht verfügbaren Raneykatalysatoren IPDA in guter Ausbeute gewonnen werden kann. Die reduktive Aminierung sollte vorzugsweise in Gegenwart eines solchen Ammoniaküberschusses durchführbar sein, der eine Druckdestillation überflüssig macht. Gelöst wird die Aufgabe durch ein Verfahren zur Herstellung von 3-Aminomethyl-3,5,5-trimethylcyclohexylamin durch reduktive Aminierung von 1,3,3-Trimethyl-5-oxo-cyclohexancarbonitril (IPN) mit Ammoniak und Wasserstoff bei einem Druck von 5 bis 20 MPa und einer Temperatur von 50 bis 150 °C unter Verwendung eines Nickel- oder Kobalt-Raney-Katalysators und in Gegenwart eines organischen Lösungsmittels, das dadurch gekennzeichnet ist, daß man zusätzlich einen Cokatalysator aus der Reihe eines Salzes von Nickel, Kobalt, Yttrium oder einem Lanthanidenelement in einer Menge von 0,01 bis 0,5 Mol pro Mol Raneykatalysator verwendet.

Erfindungsgemäß kommen Raney-Nickel- und Raney-Kobalt-Katalysatoren zur Anwendung; eingeschlossen sind auch Ni- bzw.

Co-Raney-Katalysatoren, welche mit anderen Metallen, wie insbesondere Mn und Fe, dotiert sein können. Die Raneykatalysatoren lassen sich in bekannter Weise aus Legierungen von Nickel bzw. Kobalt mit Aluminium und/oder Zn, Mg, Si sowie eventuell den Dotierungselementen durch Herauslaugen des Aluminiums bzw. des Zn, Mg, Si gewinnen, wobei meistens mit Alkalilaugen gelaugt wird.

Erfindungswesentlich ist die Verwendung von Cokatalysatoren. Bevorzugt werden Salze von Ni, Co, Y, La, Ce, insbesondere von Ni und Co. Als Anion der Salze kommen solche der Mineralsäuren, insbesondere Schwefelsäure und Salzsäure, aber auch der Carbonsäuren infrage. Halogenide, insbesondere Chloride der genannten Metalle sind gut geeignet.

Die Einsatzmenge an Cokatalysatoren kann in weiten Grenzen liegen. Zweckmäßigerweise werden pro Mol Katalysator, berechnet als Co bzw. Ni, 0.01 bis 0,5 Mol, insbesondere 0,05 bis 0,2 Mol Cokatalysator eingesetzt. Die Salze können als wasserfreie oder kristallwasserhaltige Salze in Pulverform oder als Lösung oder Suspension dem Hydrieransatz zugeführt werden.

Die Hydrierung erfolgt in Gegenwart von Ammoniak. Vorteilhafterweise werden 0,25 bis 2,5 kg NH₃, insbesondere 0,5 bis 1 kg NH₃ pro kg Isophoronnitril dem Reaktionsansatz zugefügt, etwa durch Aufpressen von flüssigem Ammoniak.

Bestandteil des Reaktionsgemisches ist schließlich noch ein organisches Lösungsmittel, worunter auch ein Lösungsmittelgemisch verstanden wird. Als Lösungsmittel kommen solche in Betracht, welche eine ausreichende Lösekraft für IPN und IPDA aufweisen, so daß bei Hydriertemperatur diese Stoffe in gelöster Form vorliegen. Geeignet sind beispielsweise niedere Alkohole, insbesondere einwertige C₁- bis C₄-Alkohole, aliphatische und cycloaliphatisce Mono- und Diether, insbesondere solche mit bis zu 6 C-Atomen, ferner allgemein Kohlenwasserstoffe, wie z. B. Cyclohexan und Tolnol. Bevorzugt werden solche Lösungsmittel, welche einen Siedepunkt unter 120 °C aufweisen und problemlos vom Reaktionsgemisch nach beendeter Hydrierung abdestilliert werden können.

Die reduktive Aminierung wird bei einem Druck im Bereich von 5 bis 20 MPa, insbesondere 10 bis 15 MPa, durchgeführt. Auf das IPN, Ammoniak, Lösungsmittel Katalysator und Cokatalysator wird im Druckreaktor Wasserstoff bis zum gewünschten Druck aufgepreßt; vorzugsweise wird der Druck während der Hydrierung durch Nachpressen von H₂ aufrechterhalten. Die Hydriertemperatur betragt 50 bis 150 °C, vorzugsweise 80 bis 130 °C.

Die Hydrierung kann diskontinuierlich oder kontinuierlich erfolgen, wobei übliche Hydrierreaktoren, wie sie für Hydrierungen unter Verwendung von Suspensionskatalysatoren Einsatz finden, verwendbar sind; beispielhaft genannt seien Rührautoklaven und Schlaufenreaktoren. Die Aufarbeitung des Reaktionsgemisches nach beendeter Hydrierung erfolgt in an sich bekannter Weise. Üblich sind die Schritte Druckentlastung, Abdunsten des Ammoniaks, Abtrennen der unlöslichen Bestandteile von der Lösung, destillative Abtrennung des Lösungsmittels und fraktionierte Destillation des IPDA-Rohproduktes unter vermindertem Druck.

Durch Verwendung der erfindungsgemäßen Cokatalysatoren war es überraschenderweise möglich, Raney-Co- und Raney-Ni-Katalysatoren zur effizienten reduktiven Aminierung von Isophoronnitril zu Isophorondiamin einzusetzen. Das Verfahren läßt sich einfach durchführen; in der Regel erübrigt sich eine Druckdestillation.

### Beispiel 1

400 g (2,42 Mol) Isophoronnitril werden in einem 5 l-Autoklaven mit Begasungsrührer in 1 l Methanol und 1 l wasserfreiem Ammoniak aufgelöst. Nach Zugabe von 100 g Raney-Cobalt (enthält ca. 25 % Wasser) und 50 g Kobaltchlorid-hexahydrat als Katalysator wird mit 1200 U/min gerührt und 130 bar Wasserstoff aufgepresst. Bei Erwärmung auf 80 °C tritt Wasserstoffaufnahme auf; gegen Ende der Hydrierung wird die Temperatur auf 130 °C angehoben. Die gesamte Reaktionszeit beträgt 3-4 Stunden. Nach dem Erkalten wird der Katalysator abfiltriert und der überschüssige Ammoniak und das Methanol abdestilliert; der Rückstand wird im Vakuum destilliert.

| | |
|---|---|
| Vorlauf: | 12 g Kp_{0,1}: 30 - 65 °C |
| Hauptlauf (IPDA) : | 350 g Kp_{0,1} : 65 - 68 °C |
| Rückstand: | 13,2 g |
| Ausbeute: | 85,0% d. Th. IPDA (bez. auf IPN) |
| Produktreinheit (GC) : | ca. 98 % |

### Beispiel 2

Der Ansatz gemäß Beispiel 1 wird wiederholt, wobei jedoch 1,5 l Methanol und 1.5 l wasserfreier Ammoniak (anstelle je 1 l) zum Einsatz kommen. Die Ausbeute an IPDA beträgt 351 g (= 85,3 % d. Th.).

### Beispiel 3

Versuch wie Beispiel 1, jedoch mit 500 g (= 3,30 Mol) Isophoronnitril und nur 0,5 l wasserfreiem Ammoniak.
- Ausbeute:: 345,1 g (67,0 % d. Th.) IPDA
- Rückstand enthält Salz:: IPDA x 1/2 HCl
Nach Freisetzung mit Natronlauge konnten insgesamt 396,6 g (77 % d. Th.) IPDA isoliert werden.

### Vergleichsbeispiel

Versuch wie Beispiel 2, jedoch ohne Kobaltchloridhexahydrat als Cokatalysator. Fast keine Wasserstoffaufnahme. Keine isolierbare Ausbeute.

## Patentansprüche

1. Verfahren zur Herstellung von 3-Aminomethyl-3,5,5-trimethyl-cyclohexylamin durch reduktive Aminierung von 1,3,3-Trimethyl-5-oxo-cyclohexancarbonitril (IPN) mit Ammoniak und Wasserstoff bei einem Druck von 5 bis 20 MPa und einer Temperatur von 50 bis 150 °C unter Verwendung eines Nickel- oder Kobalt-Raney-Katalysators und in Gegenwart eines organischen Lösungsmittels,
dadurch gekennzeichnet,
daß man zusätzlich einen Cokatalysator aus der Reihe eines Salzes von Nickel, Kobalt, Yttrium oder einem Lanthanidenelement in einer Menge von 0,01 bis 0.5 Mol pro Mol Raneykatalysator verwendet.

2. Verfahren nach Anspruch 1,
dadurch gekennzeichnet,
daß man als Cokatalysator wasserfreie oder kristallwasserhaltige Salze der genannten Metalle mit Mineralsäuren, insbesondere Schwefelsäure und Salzsäure, verwendet.

3. Verfahren nach Anspruch 1 oder 2,
dadurch gekennzeichnet,
daß man ein Halogenid, insbesondere Chlorid, von Ni, Co, Y, La oder Ce verwendet.

4. Verfahren nach einem der Ansprüche 1 bis 3,
dadurch gekennzeichnet,
daß man Katalysator und Cokatalysator im Molverhältnis von 1 zu 0,05 bis 0,2 einsetzt.

5. Verfahren nach einem der Ansprüche 1 bis 4,
dadurch gekennzeichnet,
daß man pro kg IPN 0,25 bis 2.5 kg Ammoniak einsetzt.

6. Verfahren nach einem der Ansprüche 1 bis 5,
dadurch gekennzeichnet,
daß man bei einem Druck von 10 bis 15 MPa und einer Temperatur von 80 bis 130 °C hydriert.

## Claims

1. Method for the preparation of 3-aminomethyl-3,5,5-trimethylcyclohexylamine by reductive amination of 1,3,3-trimethyl-5-oxocyclohexanecarbonitrile (IPN) with ammonia and hydrogen at a pressure of from 5 to 20 mPa and at a temperature of from 50 to 150°C, using a Raney nickel or Raney cobalt catalyst and in the presence of an organic solvent,
characterised in that
in addition a cocatalyst, selected from a salt of nickel, cobalt, yttrium, or a lanthanide element, is used in a quantity of from 0.01 to 0.5 mol per mol of Raney catalyst.

2. Method according to claim 1,
characterised in that
anhydrous salts or salts containing water of crystallisation of the said metals with mineral acids, particularly sulphuric acid and hydrochloric acid, are used as cocatalyst.

3. Method according to claim 1 or 2,
characterised in that
a halide is used, particularly chloride, of Ni, Co, Y, La or Ce.

4. Method according to one of claims 1 to 3,
characterised in that
the catalyst and cocatalyst are used in the molar ratio of 1 to 0.05 up to 0.2.

5. Method according to one of claims 1 to 4,
characterised in that
from 0.25 to 2.5 kg of ammonia is used per kg of IPN.

6. Method according to one of claims 1 to 5,
characterised in that
hydrogenation is carried out at a pressure of from 10 to 15 MPa and at a temperature of from 80 to 130°C.

## Revendications

1. Procédé d'obtention de 3-aminométhyl 3,5,5-triméthylcyclohexylamine par amination réductrice du 1,3,3-triméthyl 5-oxo cyclohexane carbonitrile (IPN) avec l'ammoniac et l'hydrogène à une pression allant de 5 à 20 MPa et à une température allant de 50 à 150°C en utilisant un catalyseur au nickel de Raney ou au cobalt de Raney et en présence d'un solvant organique, caractérisé en ce que l'on utilise en supplément un co-catalyseur choisi dans la série formée d'un sel de nickel, de cobalt, d'yttrium ou d'un élément de la série des lanthanides en une quantité allant de 0,01 à 0,5 mol par mol de catalyseur de Raney.

2. Procédé selon la revendication 1, caractérisé en ce que l'on utilise comme co-catalyseur des sels anhydres ou contenant de l'eau de cristallisation des métaux cités, avec des acides minéraux en particulier l'acide sulfurique et l'acide chlorhydrique.

3. Procédé selon la revendication 1 ou 2, caractérisé en ce que l'on utilise un halogénure, en particulier un chlorure, de Ni, Co, Y, La ou Ce.

4. Procédé selon l'une des revendications 1 à 3, caractérisé en ce que l'on met en oeuvre le catalyseur et le co-catalyseur dans un rapport molaire de 1 pour 0,05 à 0,2.

5. Procédé selon l'une des revendications 1 à 4, caractérisé en ce que l'on met en oeuvre par kg d'IPN, 0,25 à 2,5 kg d'ammoniac.

6. Procédé selon l'une des revendications 1 à 5, caractérisé en ce que l'on hydrogène à une pression de 10 à 15 Mpa et à une température de 80 à 130°C.
